# EUROPEAN PATENT APPLICATION

(11) **EP 4 609 727 A1**
(43) Date of publication of application: **03.09.2025**
(21) Application number: 23882629.1
(22) Date of filing: 24.10.2023
(51) Int. Cl.: A23L 27/20, A21D 13/064, A23C 11/02, A23G 1/44, A23G 9/38, A23J 3/08, A23J 3/14, A23J 3/16, A23L 2/00, A23L 2/38, A23L 2/52, A23L 2/66, A23L 5/00, A23L 11/00, A23L 11/65

(54) **COMPOSITION FOR IMPROVING MOUTHFEEL, FOOD/BEVERAGE PRODUCT COMPOSITION AND METHOD FOR PRODUCING SAME, AND METHOD FOR IMPROVING MOUTHFEEL**

(30) Priority: 28.10.2022 JP 2022173494
(71) Applicant: Nagase Viita Co., Ltd., Okayama-shi, Okayama 702-8006 (JP)
(72) Inventor: ITO Michie, Okayama-shi, Okayama 702-8006 (JP); KAKUTA Shoji, Okayama-shi, Okayama 702-8006 (JP); NAGAYOSHI Sayaka, Okayama-shi, Okayama 702-8006 (JP)
(74) Representative: Müller-Boré & Partner Patentanwälte PartG mbB
(86) International application number: PCT/JP2023/038313
(87) International publication number: WO 2024/090424

(57) **Abstract**

The purpose of the present invention is to provide a novel means for improving the mouthfeel of a protein-containing ingredient and to provide a food/beverage product composition containing a protein-containing ingredient that has an excellent mouthfeel. Using glucosyl naringin makes it possible to improve the mouthfeel of a protein-containing ingredient.

## Description

### [Technical Field]

The present invention relates to a composition for improving a mouthfeel of a protein-containing food material, a food and drink composition containing a protein-containing food material and a method for manufacturing the same, and a method of improving the mouthfeel of a protein-containing food material.

### [Background Art]

Protein is a nutrient which is present as a component of food such as bean, egg, meat, fish, and the like, and is one of energy-producing nutrients including carbohydrates and fats. Protein is the main component constituting cells of all the animals and plants, and is important not only as a structural component of body such as muscle, organs, skin, and hair but also as a regulatory component of the body such as hormone, enzyme, and antibody.

In recent years, the high nutritional value of protein has been attracting much attention because of growing health and beauty consciousness and an accompanying increase in the number of people with exercise habits. This has led to an increase in demand for protein-containing beverages, food products, and supplements. In addition, there are concerns about future shortages of livestock meat supply due to increased demand for livestock meat as a result of global population growth.

Accordingly, there is a growing demand for meat substitutes which are alternative food products that resemble meat in terms of mouthfeel and flavor and include plant proteins such as soybean protein as a substitute for livestock meat. Proteins used in food and drink products include a variety of food-derived proteins. For example, proteins of plant origin, such as soybean and pea, and proteins of animal origin, such as whey and gelatin, are blended in a wide range of food and drink products. However, proteins of plant origin have characteristic unpleasant tastes (harsh and bitter tastes) and unpleasant odors, and may disadvantageously spoil the flavors of food products. Especially for beverages, this may result in poor mouthfeel and feeling of going down the throat, leaving unpleasant lingering sensations in the mouth and making it difficult to drink. Animal-derived proteins may also have unpleasant tastes such as distinctive astringent tastes when heated. Disadvantageously this may impair the flavors of food and drink products.

Patent Literature 1 describes a method of improving the flavor of soybean protein by adding polyphenol extracted from tea and the like as a method of reducing grassy odor (so-called soybean odor) characteristic of soybean protein as plant protein. Further, Patent Literature 2 describes a method of controlling miscellaneous flavors such as harsh taste from plant protein materials by adding polyphenolic materials.

Moreover, Patent Literature 3 describes a method of improving or masking the quality of tastes of milk-protein containing food and drink products by adding cyclodextrin. Patent Literature 4 describes a method of controlling unpleasant flavors such as milk odor by adjusting the content of milk protein, the content of polyphenol, and the range of pH to certain ranges.

Moreover, Patent Literatures 5 and 6 describe that glucosyl naringin which has excellent water solubility can confer a favorable bitter taste on food and drink compositions. However, the effects of glucosyl naringin on the mouthfeel of protein-containing food materials are not described in any of the literatures.

### [CITATION LIST]

### [Patent Literature]

Patent Literature 1: Japanese Patent Application Laid-Open No. H8-103225
Patent Literature 2: WO2020/116565
Patent Literature 3: Japanese Patent Application Laid-Open No. 2021-185860
Patent Literature 4: Japanese Patent Application Laid-Open No. 2014-113113
Patent Literature 5: Japanese Patent Application Laid-Open No. H4-13691
Patent Literature 6: Japanese Patent Application Laid-Open No. 2002-199896

### [Summary of Invention]

### [Technical Problem]

There are limited number of means of improving the mouthfeel of a protein-containing food material, and thus there has been demand for new means in order to increase the number of options available to users. An object of the present invention is to provide a novel means for improving the mouthfeel of a protein-containing food material. Another object of the present invention is to provide a food and drink composition containing a protein-containing food material and having a good mouthfeel.

### [Solution to Problem]

After intensive studies to solve the aforementioned problems, the present inventors have found that glucosyl naringin could improve the mouthfeel of a protein-containing food material. Then, the present invention has been completed. That is, the present invention provides a composition for improving the mouthfeel of a protein-containing food material, a food and drink composition containing a protein-containing food material and a method for manufacturing the same, and a method of improving the mouthfeel of a protein-containing food material, as shown below.
[1] A composition for improving a mouthfeel of a protein-containing food material, the composition comprising glucosyl naringin.
[2] The composition for improving the mouthfeel according to [1], wherein the protein includes a plant or animal milk-derived protein.
[3] A food and drink composition, comprising glucosyl naringin and a protein-containing food material.
[4] The food and drink composition according to [3], comprising 0.5×10⁻⁶ to 1.0×10⁻² parts by mass of glucosyl naringin relative to 1 part by mass of the protein.
[5] The food and drink composition according to [3] or [4], wherein the protein includes a plant or animal milk-derived protein.
[6] The food and drink composition according to any one of [3] to [5], wherein the protein includes a legume milk-derived protein and/or a milk fluid-derived protein.
[7] The food and drink composition according to any one of [3] to [6], wherein the protein includes at least one selected from the group consisting of soybean protein, pea protein, whey protein, and casein.
[8] The food and drink composition according to any one of [3] to [7], wherein the protein includes a legume-derived protein.
[9] The food and drink composition according to any one of [3] to [8], which is in a liquid state, a fluidized state, a gel state, a semisolid state, a solid state, or a powdered state.
[10] The food and drink composition according to any one of [3] to [9], wherein the protein-containing food material includes soybean milk.
[11] A method for manufacturing the food and drink composition according to any one of [3] to [10], the method comprising a step of mixing the protein-containing food material and glucosyl naringin.
[12] The method for manufacturing according to [11], wherein 0.5×10⁻⁶ to 1.0×10⁻² parts by mass of the glucosyl naringin is blended relative to 1 part by mass of the protein.
[13] A method of improving a mouthfeel of a protein-containing food material, the method comprising a step of blending glucosyl naringin with the protein-containing food material.
[14] The method according to [13], wherein the protein includes a plant or animal milk-derived protein.
[15] The method according to [13] or [14], wherein 0.5×10⁻⁶ to 1.0×10⁻² parts by mass of the glucosyl naringin is blended relative to 1 part by mass of the protein.

### [Advantageous Effects of Invention]

According to the present invention, inclusion of glucosyl naringin can improve the mouthfeel of a protein-containing food material. In particular, glucosyl naringin can effectively improve the mouthfeel of a protein-containing food material even at a low concentration of glucosyl naringin within a range where the taste quality of glucosyl naringin itself does not make any impacts on the material. Therefore, a food and drink composition easy to eat and drink which has lightened mouthfeel can be provided even when a high concentration of the protein-containing food material is included.

### [Description of Embodiments]

Hereinafter, the present invention will be described in more detail.

It is noted that "%" and "ppm" both mean a mass-based proportion.

### <Glucosyl naringin>

Naringin is a type of polyphenol found in citrus fruit, and is known as a glycoside of an aglycone naringenin. Glucosyl naringin (GN) is a substance in which glucose is added to naringin to increase water solubility. The aforementioned GN, which is known as a substance with a bitter taste, is used as an active ingredient for improving the mouthfeel of protein as specifically shown in Examples described below of the present invention. There is no particular limitation for the aforementioned GN, but it may include, for example, monoglucosyl naringin, and preferably includes 3"-α-monoglucosyl naringin (3"-α-GN) represented by the following chemical formula:

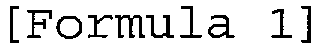

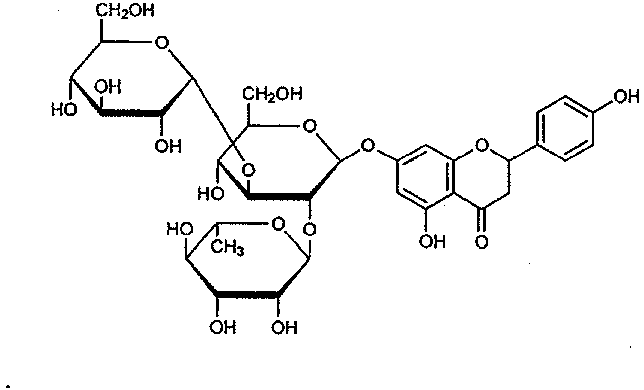

The aforementioned GN can be appropriately manufactured by a method commonly used in the art. The aforementioned GN can be manufactured, for example, by a method in which an enzyme is used, a method by chemical synthesis, a method in which fermentation is used, or combinations thereof. More specifically, a method in which glycosyltransferase is used is advantageous from an economic standpoint. For example, as described in Patent Literatures 5 and 6 and so on, when a glycosyltransferase, including α-glucosidase, cyclomaltodextrin·glucanotransferase, and α-amylase, is allowed to act on naringin in the presence of an α-glucosyl sugar compound, such as partial hydrolysates of starch and maltooligosaccharide, a series of 3"-α-glycosyl naringins having a degree of glucose polymerization at a transfer site distributed over the range between 1 and 5 can be usually obtained by high yield. By allowing glucoamylase to act on the series of 3"-α-glycosyl naringins, a composition including 3"-α-GN can be prepared. Moreover, the composition including 3"-α-GN can also be further purified to prepare highly pure 3"-α-GN.

### <Protein-containing food material>

The term "protein-containing food material" as used herein refers to a raw material or an ingredient containing edible protein, and examples of it includes both those that can be consumed directly and those that require cooking. There is no particular limitation for the aforementioned protein, and it may be plant protein or animal protein.

There is no particular limitation for the "plant protein" as used herein as long as it is derived from plant, and examples include proteins contained in legumes, cereal crops, vegetables, fruits, and the like. Legumes include, for example, soybean, pea, green soybean, fava bean, peanut, Adzuki bean, kidney bean, and the like. Cereal crops include, for example, corn, buckwheat, barley, wheat, oat, rice, and the like. Vegetables include, for example, asparagus, broccoli, cabbage, and the like. Fruits include, for example, avocado, banana, and the like. In a certain aspect, examples of the aforementioned protein include legume-derived proteins from soybean milk, tofu lees, and the like.

There is no particular limitation for the "animal protein" as used herein as long as it is derived from animal, and examples include proteins contained in meat, fish and shellfish, milk, eggs, insect, and the like. Examples of meat include livestock meat such as beef, pork, mutton, and horse meat, and poultry meat such as chicken and duck. Example of fish and shellfish include fish, shellfish, octopus, squid, shrimp, and crab. Examples of milk include mammalian milk fluid such as cow's milk, goat's milk, and sheep's milk. Examples of eggs include bird eggs such as chicken and quail eggs, and fish eggs. Examples of insect include locust, cricket, and the like.

The aforementioned plant and animal proteins may be proteins themselves derived from plant or animal sources, or may be processed proteins that have undergone physical or chemical processing of those proteins. The aforementioned processed proteins may be, for example, proteolytic products, chemically modified products of proteins, or a chemically modified products of proteolytic products.

Alternatively, the protein in the aforementioned food material may include, for example, a protein from plant or animal milk. Specifically, the aforementioned protein may include at least one selected from the group consisting of legume milk-derived proteins such as soy protein and pea protein, and milk fluid-derived proteins such as lactoserum proteins (also referred to as "whey protein" or "whey protein") and casein. In a certain aspect, the aforementioned protein-containing food material may include legume milk such as soybean milk or Pisum Sativum milk (pea milk), milk fluid, and/or whey.

### <Composition for improving mouthfeel of protein-containing food material>

The composition for improving the mouthfeel of a protein-containing food material according to the present invention includes the aforementioned GN as an active ingredient for mouthfeel improvement. The term "mouthfeel" as used herein refers to a feeling sensed when a food and drink product are placed in the mouth. The mouthfeel can be improved by adding aforementioned mouthfeel-improving composition to the aforementioned protein-containing food material, or by placing the mouthfeel-improving composition in the mouth simultaneously or sequentially with the protein-containing food material. For example, the aforementioned mouthfeel-improving composition can improve unpleasant mouthfeels (for example, heavy texture) or unpleasant flavors (for example, unpleasant tastes such as harsh, bitter, and astringent tastes, and unpleasant odors such as bean odor or milk odor) of a protein-containing food material. More specifically, the aforementioned mouthfeel-improving composition can reduce a heavy texture and unpleasant flavor of the aforementioned protein-containing food material to lighten the mouthfeel. Therefore, the aforementioned mouthfeel-improving composition is useful in processing a food and drink composition including a high concentration of the aforementioned protein-containing food material into a composition easy to eat and drink.

The aforementioned GN has a refreshingly bitter taste, but the mouthfeel-improving composition can improve the mouthfeel of the aforementioned protein-containing food material even when the aforementioned GN is used at a low concentration within a range where the bitter taste is not sensed. Without wishing to be bound by any particular theory, it is believed that the refreshing flavor of the aforementioned GN masks the unpleasant mouthfeel of the aforementioned protein-containing food material (for example, a heavy texture, unpleasant taste, unpleasant odor, and the like).

The mouthfeel-improving composition according to the present invention can be used in order to prepare a food and drink composition containing a protein-containing food material. There is no particular limitation for the usage amount of the aforementioned mouthfeel-improving composition as long as the mouthfeel-improving effect on the aforementioned protein-containing food material can be demonstrated, but, for example, the aforementioned mouthfeel-improving composition may be used in an amount so that the blending amount of the aforementioned GN is about 60 ppm or less, about 40 ppm or less, about 20 ppm or less, about 15 ppm or less, or about 10 ppm or less relative to the total mass of the aforementioned food and drink composition, or may be used in an amount so that the blending amount of the aforementioned GN is about 0.1 ppm or more, about 0.5 ppm or more, about 1 ppm or more, about 3 ppm or more, or about 5 ppm or more. More specifically, the aforementioned mouthfeel-improving composition may be used in an amount so that the blending amount of the aforementioned GN is about 0.1 ppm to about 60 ppm, about 0.1 ppm to about 40 ppm, about 0.1 ppm to about 20 ppm, about 0.1 ppm to about 15 ppm, about 0.1 ppm to about 10 ppm, about 1 ppm to about 10 ppm, or about 3 ppm to about 10 ppm relative to the total mass of the aforementioned food and drink composition. Alternatively, the aforementioned mouthfeel-improving composition may be used in an amount so that the blending amount of the aforementioned GN is about 0.5×10⁻⁶ to about 1×10⁻² parts by mass, about 1×10⁻⁶ to about 5×10⁻³ parts by mass, or about 0.5×10⁻⁵ to about 1×10⁻³ parts by mass relative to 1 part by mass of the protein in the aforementioned protein-containing food material, or may be used in an amount so that the blending amount of the aforementioned GN is about 1×10⁻⁶ to about 1×10⁻³ parts by mass, about 0.5×10⁻⁵ to about 5×10⁻⁴ parts by mass, about 0.1×10⁻⁴ to about 3.5×10⁻⁴ parts by mass, about 0.1×10⁻⁴ to about 3.04×10⁻⁴ parts by mass, about 0.41×10⁻⁴ to about 2.05×10⁻⁴ parts by mass, or about 0.46×10⁻⁴ to about 2.05×10⁻⁴ parts by mass. It is noted that the numerical ranges represented by "to" include the upper and lower limits thereof.

There is no particular limitation for the form of the mouthfeel-improving composition according to the present invention, but it may be, for example, a solid form such as a powder form, a granular form, and a block form; a liquid form; or the like. Moreover, the aforementioned mouthfeel-improving composition may be distributed in a concentrated liquid form and diluted at the time of use, or may be distributed in a powder form and dissolved at the time of use.

The mouthfeel-improving composition according to the present invention may further include an optional raw material or additive commonly used in the art, and may further include another component effective for improvement in the mouthfeel of the aforementioned protein-containing food material as long as the purposes of the present invention are not compromised. There is no particular limitation for the aforementioned optional component or additive, but examples may include food ingredients, food additives, solvents such as water and organic solvents, emulsifiers, excipients, antioxidants, flavoring components (synthetic flavoring agents, natural flavoring agents, natural essential oils, and plant extracts), or the like. Moreover, the aforementioned mouthfeel-improving composition may further include unreacted raw materials (such as naringin) when the aforementioned GN is prepared, and other naringenin glycosides as by-products.

### <Food and drink compositions

The food and drink composition according to the present invention may contain the aforementioned GN and the aforementioned protein-containing food material. The aforementioned food and drink composition may be a food and drink product to be consumed directly; a food and drink product to be treated before consumption; or a mixture of ingredients, work-in-process, or seasoning for preparing different food and drink products; and the like. The aforementioned GN has an effect for improving the mouthfeel of the aforementioned protein-containing food material, and thus the food and drink composition or a food and drink product prepared using that food and drink composition may have a lighter mouthfeel than a food and drink composition having the same composition except that it does not contain the aforementioned GN.

There is no particular limitation for the ratio of the content of the aforementioned GN and the content of protein in the food and drink composition according to the present invention, but it may be, for example, about 0.5×10⁻⁶ to about 1×10⁻² parts by mass, about 1×10⁻⁶ to about 5×10⁻³ parts by mass, or about 0.5×10⁻⁵ to about 1×10⁻³ parts by mass relative to 1 part by mass of the protein, or may be about 1×10⁻⁶ to about 1×10⁻³ parts by mass, about 0.5×10⁻⁵ to about 5×10⁻⁴ parts by mass, about 0.1×10⁻⁴ to about 3.5×10⁻⁴ parts by mass, about 0.1×10⁻⁴ to about 3.04×10⁻⁴ parts by mass, about 0.41×10⁻⁴ to about 2.05×10⁻⁴ parts by mass, or about 0.46×10⁻⁴ to about 2.05×10⁻⁴ parts by mass.

There is no particular limitation for the content of protein in the food and drink composition according to the present invention, but it may be, for example, about 1 mass% or more, about 2 mass% or more, about 3 mass% or more, about 4 mass% or more, or about 5 mass% or more relative to the total mass of the aforementioned food and drink composition, or may be about 40 mass% or less, 20 mass% or less, or 10 mass% or less.

There is no particular limitation for the concentration of the aforementioned GN in the food and drink composition according to the present invention, but it may be, for example, about 60 ppm or less, about 40 ppm or less, about 20 ppm or less, about 15 ppm or less, or about 10 ppm or less relative to the total mass of the aforementioned food and drink composition, or may be about 0.1 ppm or more, about 0.5 ppm or more, about 1 ppm or more, about 3 ppm or more, or about 5 ppm or more. More specifically, it may be about 0.1 ppm to about 60 ppm, about 0.1 ppm to about 40 ppm, about 0.1 ppm to about 20 ppm, about 0.1 ppm to about 15 ppm, about 0.1 ppm to about 10 ppm, about 1 ppm to about 10 ppm, or about 3 ppm to about 10 ppm. When the concentration of the aforementioned GN falls within these ranges, a food and drink composition can be prepared in which the mouthfeel of the aforementioned protein-containing food material is effectively improved without being affected by the taste quality of the aforementioned GN.

There is no particular limitation for the form of the aforementioned protein-containing food material used for the food and drink composition according to the present invention, but, for example, a purified protein material prepared from a protein-containing raw material may be used. More specifically, the aforementioned purified protein material may include a concentrated protein material which is prepared by removing components other than the desired protein from the raw material, a separated protein material which is prepared by retrieving the desired protein from a raw material, or the like. There is no particular limitation for the form of the aforementioned protein-containing food material, but it may be, for example, a powder form, a granular form, or a particulate form, and may be suitably selected according to the type of the aforementioned food and drink composition.

There is no particular limitation for the form of the food and drink composition according to the present invention, but it may be, for example, a flowable form (including a liquid form, a syrup form, an emulsified form, and suspended from, whether viscus or not), a gel form, a paste form, a semisolid form, a solid form, a powder form, or the like. Specifically, the aforementioned food and drink composition may be, for example, alcoholic beverages such as synthetic Sake, brewed Sake, refined Sake, fruit liquor, low-malt beer, beer, liqueur, Shochu highball, and medicinal liquor; drinks such as carbonated drinks, soft drinks, tonic water, dairy drinks, almond milk, artificial milk for infants, protein drinks, smoothies, vegetable juices, fruit juices, sports drinks, vinegar drinks, iron-containing drinks, lactobacillus drinks, green tea, black tea, herbal tea, cocoa, coffee, non-alcoholic drinks, and nutrition-supplement drinks; staple diets such as rice, rice porridge, rice cake, and bread; noodles such as Udon noodles, buckwheat noodles, Ramen noodles, and spaghetti; soups such as miso soup, Osuimono soup, and vegetable soup; products made from soybean protein or other soy distillates such as soybean milk products, beverages containing separated or enzyme-treated soybean protein, soybean flour-containing beverages, soybean lecithin-containing products, tofu, and fermented food products such as tempeh; dairy products such as yogurt, cheese, cream cheese, coffee whitener, dried milk powder, butter, and buttermilk; livestock meat products such as sausage and ham; egg products such as dried egg, egg white, and egg yolk; grain products such as cereals, muesli bars, and cooked and ready-to-consume rice products; fish meat products such as Kamaboko, Chikuwa, Hanpen, and fish sausage; processed seafood products such as canned fish and shellfish and dried fish; pickles such as lightly-pickled vegetables, pickled Japanese radish, Kimchi, pickled plum, and Shibazuke pickles; confectionery such as soft candy, hard candy, gummi candy, jelly, cooky, soft cooky, rice cracker, Gyuhi, rice cake, mousse, bavarois, biscuit, chocolate, chewing gum, caramel, fruit paste, jam, marmalade, cereal bars, protein bars, and energy bars; frozen confectionery such as ice cream, sherbet, and gelato; or various seasonings and cooked or semi-cooked processed food products (including frozen food products) such as miso paste, powdered miso, soy sauce, powdered soy sauce, mayonnaise, dressing, edible vinegar, noodle soup base, sauce, tomato sauce, pasta sauce, curry roux, soup base, Ramen soup, and mixed seasoning. The aforementioned cooked or semi-cooked processed food products may be a food product of the particular application such as care diet, hospital diet, patient diet, medical diet, and fluid diet. Moreover, the aforementioned food and drink composition may be a protein bar, a protein supplement, powdered protein drink, soft drink, soup, heat-and-serve meal, confectionery, and chilled confectionery, which contain a relatively large amount of multiple types of proteins for the purposes of nutritional support or enhancement.

In a certain aspect, the aforementioned protein-containing food material includes soybean milk. There is no particular limitation for the aforementioned food and drink composition in this case, but it may be suitably prepared in a form of a food and drink product prepared using soybean milk, for example, soybean milk beverage, soybean milk soup, soybean protein beverage, soybean milk cheese, soybean milk margarine, soybean milk bar, soybean milk sauce, soybean milk jelly, soybean milk pudding, soybean milk ice cream, soybean milk yogurt, tofu, and the like.

The food and drink composition according to the present invention may further include an optional raw material or additive commonly used in the art, and may further include another component effective for improvement in the mouthfeel of the aforementioned protein-containing food material as long as the purposes of the present invention are not compromised. There is no particular limitation for the aforementioned optional raw material or additive, but it may include, for example, a sweetener, an acidulant, a bittering agent, a seasoning, a flavoring agent, a polysaccharide thickener, an emulsifying agent, an antiseptic agent, a bactericidal or antimicrobial agent, a pH adjuster, an tonicity agent, a chelating agent, a stabilizing agent, an antioxidizing agent, a coloring agent, a bulking agent, a flow improver, an excipient, a bonding agent, a disintegrating agent, a solvent, a softener, an oil, a filler, a foaming agent, an antifoaming agent, a nutriment, a favorite material, a taste material, or a medicinal material. Moreover, the aforementioned food and drink composition may further include unreacted raw materials (such as naringin) when the aforementioned GN is prepared, and other naringenin glycosides as by-products.

### <Method for manufacturing food and drink compositions

The method for manufacturing a food and drink composition according to the present invention comprises a step of mixing the aforementioned protein-containing food material and the aforementioned GN. Various raw materials including the aforementioned protein-containing food material and the aforementioned GN can be suitably mixed and optionally processed to manufacture the food and drink composition described above in one aspect of the present invention. There is no particular limitation for the order of blending the aforementioned protein-containing food material and the aforementioned GN. When another raw material is present, one of the aforementioned protein-containing food material and the aforementioned GN may be first added to that another raw material, and then the other may be added, or they may be added simultaneously.

### <Method of improving mouthfeel of protein-containing food material>

The method of improving the mouthfeel of a protein-containing food material according to the present invention comprises a step of blending the aforementioned GN with the aforementioned protein-containing food material. The aforementioned GN can show an effect for improving the mouthfeel of the aforementioned protein-containing food material. Therefore, the mouthfeel of a food and drink composition including the aforementioned protein-containing food material will be improved. The aforementioned protein-containing food material and the aforementioned GN are as described above with respect to the mouthfeel-improving agent for the protein-containing food material and the food and drink composition according to one aspect of the present invention.

Unless the purposes of the present invention are impaired, the method of improving the mouthfeel of a protein-containing food material according to the present invention may further include an optional step commonly used in the art, or may further include a step of adding another component effective for improving the mouthfeel of a protein-containing food material.

Although the present invention will be described in more detail with reference to the following Examples, the present invention shall not be limited to these Examples, but rather can be varied and modified as appropriate without departing from the technical scope of the present invention.

### [Examples]

### <Experiment 1: Preparation and analysis of 3"-α-monoglucosyl naringin>

Naringin was sequentially treated with enzymes according to the method of preparing the C liquid according to Example 1 of Patent Literature 6 (Japanese Patent Application Laid-Open No. 2002-199896) to prepare a composition including 3"-α-monoglucosyl naringin (3"-α-GN) (hereinafter also referred to as "enzyme-treated naringin") . The content of 3"-α-GN in the resulting composition was determined under the HPLC analysis conditions as shown below, and the content of 3"-α-GN content was found to be about 70% with the remainder being unreacted naringin and other substances.

### - HPLC analysis conditions

Column: "CAPCELL PAK C18 UG 120" (from Shiseido Co., Ltd)
Eluent: water/acetonitrile/acetic acid = 80/20/0.01 (v/v/v)
Detection: UV 280 nm
Temperature: 40°C
Flow rate: 0.8 mL/min

In order to increase the purity of 3"-α-GN, the enzyme-treated naringin was dissolved in a mixed liquid of water/acetonitrile/acetic acid (80/20/0.01 (v/v/v)), and allowed to pass through the C18 column using a method in accordance with the aforementioned HPLC analysis conditions, i.e., using an ultraviolet light absorption spectrometer (UV 280 nm) as a detector to separate 3"-α-GN from naringin, and the fractions of 3"-α-GN were collected. Then, these fractions were concentrated under reduced pressure, and powdered by spray drying to obtain a powdered highly pure 3"-α-GN. This highly pure 3"-α-GN was washed with pure water, and freeze-dried. This was repeated twice to obtain an authentic sample of 3"-α-GN for use in the following experiments and preparation examples. This authentic sample of 3"-α-GN were analyzed by the HPLC method as described above to find that the purity was 99.0 mass%.

### <Experiment 2: Mouthfeel-improving effect of 3"-α-GN on protein-containing food material (1)>

In order to evaluate the mouthfeel-improving effect of 3"-α-GN on a protein-containing food material, the authentic sample of 3"-α-GN prepared in Experiment 1, an enzyme-treated naringin composition (Product name "NARINVID" (registered trademark), the content of 3"-α-GN: 70%, available from Hayashibara Co., Ltd.), or naringin (available from Sichuan Xinhuakang Biological Technology Co., Ltd., the purity: 98.2%) was added to a solution of soybean protein in which powdered soybean protein derived from soybean milk (Product Name "Fujipro CLE", the content of protein: 86.3%, available from Fuji Oil Co., Ltd.) was dissolved in water to give the concentrations shown in Table 1 below. Thereby, soybean protein liquids of the control sample 1 and the test samples 1-1 to 1-3 were prepared.

### [Table 1]

**Table 1**

| | Control sample 1 | Test samples | | |
|---|---|---|---|---|
| | | 1-1 | 1-2 | 1-3 |
| 0.1% solution of enzyme-treated naringin (mL) | 0 | 4.6 | 0 | 0 |
| 0.1% solution of authentic sample of 3"-α-GN (mL) | 0 | 0 | 4.6 | 0 |
| 0.1% solution of naringin (mL) | 0 | 0 | 0 | 4.6 |
| Solution of powdered soybean protein (g) | 460 | 460 | 460 | 460 |
| Water (mL) | 4.6 | 0 | 0 | 0 |
| Content of GN (ppm) | 0 | 7 | 10 | 0 |
| Content of protein (%) | 8.5 | 8.5 | 8.5 | 8.5 |
| GN/P (×10⁻⁴) *1 | - | 0.82 | 1.18 | - |

| | | | | |
|---|---|---|---|---|
| *1 The mass ratio (GN/P) of the amount of GN (GN) to the amount of protein (P). | | | | |

Then, 10 panelists with food sensory evaluation skills who had passed the five-taste test (hereinafter referred to as "expert panelists") conducted sensory evaluation tests on the test samples. Specifically, each of the expert panelists sequentially placed the control sample and the test samples at room temperature into the mouth, and evaluated the presence or absence of improvement in the "heavy texture" and "harsh taste of soybean" of the test samples as compared to the control sample, and recorded their findings about tastes and odors. The term "heavy texture" refers to the degree of heaviness of mouthfeel and aftertaste of a samples when the sample is placed in the mouth. In this test, the presence or absence of improvement in the heaviness of mouthfeel and aftertaste when the samples were placed in the mouth was evaluated with the reference to those sensed when the control sample was placed in the mouth. The term "harsh taste of soybean" refers to the degree of distinctive bean odor and unpleasant lingering taste which remains on the back of the tongue and the mucous membrane of the mouth when placed in the mouth. In this test, the presence or absence of improvement in the unpleasantness when the samples were placed in the mouth was evaluated with the reference to that sensed when the control sample was placed in the mouth. Specifically, the following criteria were used to evaluate the "heavy texture" and "harsh taste of soybean." When more than half of the panelists evaluated a test sample as "+" that sample was determined to have improved the mouthfeel (Good), or otherwise determined to have no effect on the mouthfeel (Bad). The results are shown in Table 2.

### "Heavy texture"

+: lighter and more refreshing mouthfeel than the control sample
±: as heavy texture as the control sample
-: heavier mouthfeel and more lingering aftertaste than the control sample

### "Harsh taste of soybean"

+: weaker than the control sample
±: similar to the control sample
-: stronger than the control sample

### [Table 2]

**Table 2**

| | Test samples | | |
|---|---|---|---|
| | 1-1 | 1-2 | 1-3 |
| Heavy texture | | | |
| + | 9 | 7 | 3 |
| ± | 0 | 3 | 5 |
| - | 1 | 0 | 2 |
| Evaluation | Good | Good | Bad |

| Harsh taste of soybean | | | |
|---|---|---|---|
| + | 6 | 8 | 2 |
| ± | 1 | 1 | 5 |
| - | 3 | 1 | 3 |
| Evaluation | Good | Good | Bad |

As shown in Table 2, the test samples 1-1 and 1-2 including 3"-α-GN showed improved "heavy texture" and "harsh taste of soybean" of the solutions of soybean protein as compared to the control sample. The similar level of improvement in mouthfeel was observed in both of the test samples. Moreover, these test samples had more refreshing mouthfeel than the control sample and showed neither harsh taste nor bitter taste. They were easy to drink. In contrast, the test sample 1-3, which included naringin, a type of polyphenol glycoside, did not show improvement in the mouthfeel of the solution of soybean protein. The test sample 1-3 was also found to have bitter taste. These results indicate that 3"-α-GN has an effect of improving the mouthfeel of a protein-containing food material, and also suggest that this effect may be a unique effect not found in other polyphenol glycosides.

### <Experiment 3: Mouthfeel-improving effect of 3"-α-GN for improving mouthfeel on soybean protein (2)>

The same tests were performed as in Experiment 2 except that the concentrations of 3"-α-GN were altered. That is, powdered soybean protein derived from soybean milk (Product Name "Fujipro CLE", the content of protein: 86.3%, available from Fuji Oil Co., Ltd.) was dissolved in water to give a concentration of 2 mass%, and stirred under heating, and then subjected to high-pressure homogenization. To this solution of soybean protein, added was enzyme-treated naringin (Product name "NARINVID" (registered trademark), the content of 3"-α-GN: 70%, available from Hayashibara Co., Ltd.) to give a concentration of 1 ppm or 5 ppm (0.7 ppm or 3.5 ppm of 3"-α-GN), or added was the same amount of water. Thereby, the test samples 2-1 and 2-2 and the control samples 2-1 and 2-2 having compositions listed in Table 3 were prepared. Then, 9 expert panelists conducted sensory evaluation tests on each of the test samples as in Experiment 2. The results are shown in Table 4.

### [Table 3]

**Table 3**

| | Control samples | | Test samples | |
|---|---|---|---|---|
| | 2-1 | 2-2 | 2-1 | 2-2 |
| 1% solution of enzyme-treated naringin (mL) | 0 | 0 | 0.025 | 0.125 |
| Powdered soybean protein (g) | 5 | 5 | 5 | 5 |
| Water (mL) | 245.025 | 245.125 | 245 | 245 |
| Content of GN (ppm) | 0 | 0 | 0.7 | 3.5 |
| Content of protein (%) | 1.7 | 1.7 | 1.7 | 1.7 |
| GN/P (×10⁻⁴) *1 | - | - | 0.41 | 2.06 |

| | | | | |
|---|---|---|---|---|
| *1 The mass ratio (GN/P) of the amount of GN (GN) to the amount of protein (P). | | | | |

### [Table 4]

**Table 4**

| | Test samples | |
|---|---|---|
| | 2-1 | 2-2 |
| Heavy texture | | |
| + | 5 | 5 |
| ± | 3 | 2 |
| - | 1 | 2 |
| Evaluation | Good | Good |

| Harsh taste of soybean | | |
|---|---|---|
| + | 6 | 5 |
| ± | 2 | 3 |
| - | 1 | 1 |
| Evaluation | Good | Good |

As shown in Table 4, both in the test sample 2-1 which included 3"-α-GN at a concentration lower than that used in Experiment 2 relative to the protein and in the test sample 2-2 which included 3"-α-GN at a concentration higher than that used in Experiment 2, improvement effects on the "heavy texture" and harsh taste of soybean" were observed. Moreover, the bitter taste from 3"-α-GN was not sensed in all the test samples.

### <Experiment 4-1: Mouthfeel-improving effect of 3"-α-GN on soybean milk (1)>

To 1,000 mL of plain soybean milk (Product name: "Tokusei-Noukou 14.0 plain soybean milk", the content of soybean solid: 14%, the content of protein: 6.8%, available from MARUSAN-AI CO., LTD.), added was enzyme-treated naringin (Product name "NARINVID" (registered trademark), the content of 3"-α-GN: 70%, available from Hayashibara Co., Ltd.) so that the concentration of 3"-α-GN was 0.7 ppm, or added was the same amount of water as these. Thereby, the test sample 3 and the control sample 3 having compositions listed in Table 5 were prepared.

### [Table 5]

**Table 5**

| | Control sample 3 | Test sample 3 |
|---|---|---|
| 1% solution of enzyme-treated naringin (mL) | 0 | 0.1 |
| Plain soybean milk (mL) | 1000 | 1000 |
| Water (mL) | 2.0 | 1.9 |
| Content of GN (ppm) | 0 | 0.7 |
| Content of protein (%) | 6.8 | 6.8 |
| GN/P (×10⁻⁴) *1 | - | 0.1 |

| | | |
|---|---|---|
| *1 The mass ratio (GN/P) of the amount of GN (GN) to the amount of protein (P). | | |

The test sample 3 was subjected to the sensory evaluation test by 15 expert panelists as in Experiment 2. The results showed that 10 out of the 15 panelists determined that the test sample 3 had lighter and more refreshing mouthfeel than the control sample 3, demonstrating the mouthfeel-improving effect of 3"-α-GN on soybean milk.

Further, the test sample 3 also showed a trend toward improvement in the harsh taste of soybean. For the separately prepared test samples in which the concentration of 3"-α-GN was increased to 3.5 ppm or 7 ppm, 8 out of the 15 expert panelists determined that the harsh taste of soybean was weaker. These results more clearly demonstrated the improving effect. In addition, no bitter taste was sensed at a concentration of 3"-α-GN in the range of 0.7 ppm to 7 ppm, indicating that the effect for improving the mouthfeel of a protein-containing food material can be shown at or below the threshold for perception of the bitter taste.

### <Experiment 4-2: Mouthfeel-improving effect of 3"-α-GN on soybean milk (2)>

Samples of soybean milk having different contents of soybean solid were used to perform the tests as in Experiment 4-1. That is, to 900 mL of plain soybean milk (Product name: "Tohu Mo Dekimasu - Organic soybean milk", the content of soybean solid: 10%, the content of protein: 8.6%, available from Nagoya Meiraku Co., Ltd.), added was enzyme-treated naringin (Product name: "NALINVID" (registered trademark), the content of 3"-α-GN: 70%, available from Hayashibara Co., Ltd.) to give a concentration of 10 ppm (the concentration of 3"-α-GN was 7 ppm.), or added was the same amount of water as these. Thereby, the test sample 4 and the control sample 4 were prepared having compositions listed in Table 6.

### [Table 6]

**Table 6**

| | Control sample 4 | Test sample 4 |
|---|---|---|
| 1% solution of enzyme-treated naringin (mL) | 0 | 0.9 |
| Plain soybean milk (mL) | 900 | 900 |
| Water (mL) | 0.9 | 0 |
| Content of GN (ppm) | 0 | 7 |
| Content of protein (%) | 5 | 5 |
| GN/P (×10⁻⁴) *1 | - | 1.4 |

| | | |
|---|---|---|
| *1 The mass ratio (GN/P) of the amount of GN (GN) to the amount of protein (P). | | |

The test sample 4 was subjected to the sensory evaluation test by 10 expert panelists as in Experiment 2. The results showed that 6 out of the 10 panelists determined that the test sample 4 had lighter and more refreshing mouthfeel than the control sample 4, demonstrating the mouthfeel-improving effect of 3"-α-GN on the samples of soybean milk having different contents of soybean solid. Further, 5 out of the 10 panelists determined that the test sample 4 had weaker harsh taste of soybean than the control sample 4. In addition, the bitter taste from 3"-α-GN was not sensed in the test sample 4.

The results from Experiment 4-1 and Experiment 4-2 indicated that 3"-α-GN showed the effect for improving mouthfeel of the samples of soybean milk having relatively large soybean solid contents of 10% and 14%. This suggested that even a food and drink composition having a relatively large protein content which has a heavy texture or unpleasant taste characteristic of protein-containing food materials can be improved in terms of mouthfeel by adding the aforementioned GN, leading to a food and drink products which are easy to eat and drink.

### <Experiment 5: Mouthfeel-improving effect on high-protein content tofu>

A soybean milk liquid having a composition as shown in Table 7 below was prepared. This soybean milk liquid was used to produce high-protein content tofu. Specifically, 43 g of soybean milk-derived powdered soybean protein (Product name "Fujipro CLE", the content of protein: 86.3%, available from Fuji Oil Co., Ltd.) was added to 957 g of plain soybean milk (Product name "Tokunou soybean milk, plain", the content of soy solid: 11%, the content of protein: 5.2%, available from CGC Japan Co., Ltd.), and stirred with a homogenizer at 5,000 rpm for 10 minutes at room temperature, and stored in a refrigerator overnight. Thereby, 1,000 g of a high-protein soybean milk liquid was obtained. To 1,000 g of the resulting high-protein soybean milk liquid, added were 1 g of 1% enzyme-treated naringin solution (the content of 3"-α-GN: 70%) and 27 g of water, which was stirred and then cooled. Then, 15 g of bittern was added, and then filled into a container and heated in a steam convection oven at 90°C for 45 minutes. After heating, it was stored in a refrigerator overnight to obtain a GN-containing high-protein content tofu (the test tofu) (the content of protein: 8.3%). A control tofu with a high protein content (the control tofu) was also prepared in similar way except that water was used instead of 1% solution of enzyme-treated naringin.

### [Table 7]

**Table 7**

| | Control tofu | Subject tofu |
|---|---|---|
| High-protein soybean milk liquid (g) | 1000 | 1000 |
| 1% solution of enzyme-treated naringin (mL) | 0 | 1 |
| Bittern (g) | 15 | 15 |
| Water (g) | 28 | 27 |
| Content of GN (ppm) | 0 | 7 |
| Content of protein (%) | 8.3 | 8.3 |
| GN/P (×10⁻⁴) *1 | - | 0.84 |

| | | |
|---|---|---|
| *1 The mass ratio (GN/P) of the amount of GN (GN) to the amount of protein (P). | | |

The test tofu was subjected to the sensory evaluation test by 15 expert panelists as in Experiment 2. The results showed that 8 out of the 15 panelists determined that the test tofu had lighter and more refreshing mouthfeel than the control tofu, and that 8 out of the 15 panelists determined that the test tofu had weaker harsh taste of soybean than the control tofu, demonstrating the mouthfeel-improving effect of 3"-α-GN even on the form of tofu. In addition, the bitter taste from 3"-α-GN was not sensed in the test tofu. In addition to the "heavy texture" and the "harsh taste of soybean, the "powderiness" which represents a degree of powderiness sensed on the tongue and the throat when the sample is placed in the mouth, was also evaluated. The results showed that the test tofu had a trend toward improvement in the powderiness more than the control tofu.

### <Experiment 6: Effects of 3"-α-GN for improving mouthfeel of lactoserum protein>

Tests were conducted to evaluate the effect of 3"-α-GN for improving the mouthfeel of lactoserum protein. Specifically, a powdered whey protein (Product name "FIXIT THINK SIMPLE", the content of protein: 76.3%, available from G.O. Holdings Co., Ltd.) as a protein powder was dissolved in water to give concentrations of 3 mass%, 5 mass%, 10 mass%, or 20 mass%, and stirred well. Then, to each of the protein solutions, added was enzyme-treated naringin (Product name "NARINVID" (registered trademark), the content of 3"-α-GN: 70%, available from Hayashibara Co., Ltd.) to give a concentration of 10 ppm, or added was the same amount of water as this. Thereby, the test samples 6-1 to 6-4 and the control samples 6-1 to 6-4 having compositions listed in Table 8 were obtained.

### [Table 8]

**Table 8**

| | Control sample | | | | Test samples | | | |
|---|---|---|---|---|---|---|---|---|
| | 6-1 | 6-2 | 6-3 | 6-4 | 6-1 | 6-2 | 6-3 | 6-4 |
| 1% solution of enzyme-treated naringin (mL) | 0 | 0 | 0 | 0 | 0.3 | 0.3 | 0.3 | 0.3 |
| Powdered whey protein (g) | 9 | 15 | 30 | 60 | 9 | 15 | 30 | 60 |
| Water (mL) | 291.3 | 285.3 | 270.3 | 240.3 | 291 | 285 | 270 | 240 |
| Content of GN (ppm) | 0 | 0 | 0 | 0 | 7 | 7 | 7 | 7 |
| Content of protein (%) | 2.3 | 3.8 | 7.6 | 15.3 | 2.3 | 3.8 | 7.6 | 15.3 |
| GN/P (×10⁻⁴) *1 | - | - | - | - | 3.04 | 1.84 | 0.92 | 0.46 |

| | | | | | | | | |
|---|---|---|---|---|---|---|---|---|
| *1 The mass ratio (GN/P) of the amount of GN (GN) to the amount of protein (P). | | | | | | | | |

Each of the test samples was subjected to the sensory evaluation tests by 6 expert panelists as in Experiment 2 except that "milk odor" unpleasant odor from whey protein was evaluated instead of the "harsh taste of soybean". The results are shown in Table 9.

### "Milk odor"

+: weaker than the control sample
±: similar to the control sample
-: stronger than the control sample

### [Table 9]

**Table 9**

| | Test samples | | | |
|---|---|---|---|---|
| | 6-1 | 6-2 | 6-3 | 6-4 |
| Heavy texture | | | | |
| + | 6 | 4 | 6 | 5 |
| ± | 0 | 1 | 0 | 1 |
| - | 0 | 1 | 0 | 0 |
| Evaluation | Good | Good | Good | Good |

| Milk odor | | | | |
|---|---|---|---|---|
| + | 5 | 3 | 6 | 3 |
| ± | 1 | 3 | 0 | 1 |
| - | 0 | 0 | 0 | 2 |
| Evaluation | Good | Good | Good | Good |

As shown in Table 9, the "heavy texture" and the "milk odor" of the solutions of whey protein was improved for the test samples 6-1 to 6-4 including 3"-α-GN as compared to the control sample, demonstrating the mouthfeel-improving effect of 3"-α-GN. Moreover, these test samples all had more refreshing mouthfeel than the control samples, and showed neither harsh taste nor bitter taste, and were easy to drink.

### <Experiment 7: Mouthfeel-improving effect of 3"-α-GN on pea protein>

Tests were conducted to evaluate the effect of 3"-α-GN for improving the mouthfeel of pea protein. Specifically, a powdered pea protein derived from pea milk (Product name "PeaProtein hydrolyzed", the content of protein: 80.0%, available from YanTai Shuangta Food Company Ltd.) as powdered protein was added in water to give a concentration of 10 mass%, and homogenized at 2,500 rpm while heated in a thermostatic bath at 60°C. Then, this was subjected to a high-pressure homogenizer (20 MPa) to prepare a solution of pea protein. To the prepared protein solution, added was enzyme-treated naringin (Product name "NARINVID" (registered trademark), the content of 3"-α-GN: 70%, available from Hayashibara Co., Ltd.) to give a concentration of 10 ppm (7 ppm of 3"-α-GN), or added was the same amount of water as this. Thereby, the test sample 7 and the control sample 7 having compositions listed in Table 10.

### [Table 10]

**Table 10**

| | Control sample 7 | Test sample 7 |
|---|---|---|
| 1% solution of enzyme-treated naringin (mL) | 0 | 1 |
| Powdered peas protein (g) | 100 | 100 |
| Water (mL) | 901 | 900 |
| Content of GN (ppm) | 0 | 7 |
| Content of protein (%) | 8 | 8 |
| GN/P (×10⁻⁴) *1 | - | 0.88 |

| | | |
|---|---|---|
| *1 The mass ratio (GN/P) of the amount of GN (GN) to the amount of protein (P). | | |

The test sample 7 was subjected to the sensory evaluation tests by 10 expert panelists as in Experiment 2, except that "pea odor" unpleasant odor from pea protein was evaluated instead of "harsh taste of soybean".

### "Pea odor"

+: weaker than the control sample
±: similar to the control sample
-: stronger than the control sample

The results showed that 9 out of the 10 panelists determined that the test sample 7 had lighter and more refreshing mouthfeel than the control sample 7, demonstrating the mouthfeel-improving effect of 3"-α-GN even on the pea protein. Further, 9 out of the 10 panelists determined that that the pea odor was weaker in the test sample 7 than in the control sample 7. In addition, the bitter taste from 3"-α-GN was not sensed in the test sample 7.

### <Preparation Example>

### (1) Soybean milk

To 900 parts by mass of commercially available plain soybean milk (Product name: "Tohu Mo Dekimasu - Organic soybean milk", the content of soybean solid: 10%, the content of protein: 5%, available from Nagoya Meiraku Co.,Ltd.), added was enzyme-treated naringin (Product name: "NARINVID" (registered trademark), the content of GN: 70%, available from Hayashibara Co., Ltd.) to give a concentration of 5 ppm. The resulting soybean milk (the concentration of 3"-α-GN: 3.5 ppm, the content of soybean solid: 10%, the content of protein: 5%) had lighter mouthfeel and clean refreshing aftertaste as compared to the control soybean milk having water in place of the enzyme-treated naringin, and was easy to drink with reduced bitter taste characteristic of soybean. Further, the bitter taste from 3"-α-GN was not sensed.

### (2) Soybean milk beverage

To the mixture of 500 parts by mass of commercially available plain soybean milk (Product name "Delicious plain soybean milk", available from Kikkoman Corporation, the content of soybean solid: 8%, the content of protein: 4.1%) and 500 parts by mass of water, added was an enzyme-treated naringin composition (Product name "NARINVID" (registered trademark), the content of 3"-α-GN: 70%, available from Hayashibara Co., Ltd.) to give a concentration of 5 ppm. The resulting soybean milk beverage (the concentration of 3"-α-GN: 3.5 ppm, the content of soybean solid: 4%, the content of protein: 2.1%) had light mouthfeel, and was easy to drink with reduced harsh taste and soybean odor characteristic of soybean milk beverages. Further, the bitter taste from 3"-α-GN was not sensed.

### (3) Powdered soybean protein beverage

Mixed were 3.0 parts by mass of soybean protein powder derived from soybean milk (the content of protein: 85%), 3.5 parts by mass of granulated sugar, 2.5 parts by mass of dextrose, 0.5 parts by mass of tartaric acid, 0.3 parts by mass of sodium bicarbonate, 0.1 parts by mass of anhydrous citric acid, 0.07 parts by mass of a powdered flavoring agent, 0.03 parts by mass of dye powder, to which enzyme-treated naringin (Product name "NARINVID" (registered trademark), the content of 3"-α-GN: 70%, available from Hayashibara Co., Ltd.) was added to give a concentration of 50 ppm. Thereby, a powdered soybean protein beverage was obtained. To 10 parts of this powdered soybean protein beverage, added was 90 parts of water to prepare a soybean protein beverage (the concentration of 3"-α-GN: 3.5 ppm, the content of protein 2.6%). The resulting soybean milk beverage had light mouthfeel, and was easy to drink with reduced harsh taste and soybean odor characteristic of soybean. Further, the bitter taste from 3"-α-GN was not sensed.

### (4) Protein drink

Mixed were 80.73 parts by mass of water, 9.4 parts by mass of high fructose corn syrup, 4.7 parts by mass of whey protein concentrate (the content of protein: 80%), 4.7 parts by mass of apple juice concentrate, 0.35 parts by mass of 85% solution of phosphoric acid, 0.1 parts by mass of flavoring agent, and 0.02 parts by mass of coloring agent, to which an enzyme-treated naringin composition (Product name "NARINVID" (registered trademark), the content of 3"-α-GN: 70%, available from Hayashibara Co., Ltd.) was added to give a concentration of 1 ppm. Thereby, a low-pH protein drink (the concentration of 3"-α-GN: 0.7 ppm, and the content of protein: 3.8%) was obtained. The resulting protein drink had light mouthfeel, and was easy to drink with reduced milk odor characteristic of whey protein. Further, the bitter taste from 3"-α-GN was not sensed.

### (5) Tofu

Mixed and stirred uniformly were 1000 parts by mass of soybean milk (the content of protein: 5.2%), 20 parts by mass of trehalose (Product name "TREHA", available from Hayashibara Co., Ltd.), and 10 parts by mass of bittern. To this mixture, an enzyme-treated naringin composition (Product name "NARINVID" (registered trademark), the content of 3"-α-GN: 70%, available from Hayashibara Co., Ltd.) was added to give a concentration of 10 ppm, and filled into a container, and heated at 75°C for 30 minutes, and then cooled to prepare tofu (the concentration of 3"-α-GN: 7 ppm, the content of protein: 5.0%) .The resulting tofu had light mouthfeel and was easy to eat with reduced harsh taste and odor characteristic of soybean. Further, the bitter taste from 3"-α-GN was not sensed.

### (6) Soybean milk pudding

The mixture of 6 parts by mass of carrageenan, 50 parts by mass of sugar, and 60 parts by mass of powdered whole milk was added to 880 parts by mass of soy milk diluted with water (30 parts by mass of soybean protein) with stirring, to which 4 parts by mass of vanilla flavoring was further added. To this, an enzyme-treated naringin composition (Product name "NARINVID" (registered trademark), the content of 3"-α-GN: 70%, available from Hayashibara Co., Ltd.) was added and mixed to give a concentration of 1 ppm. The resulting mixture was heated at 85°C for 5 minutes with stirring, and filled into a container, and cooled to prepare soybean milk pudding (the concentration of 3"-α-GN: 0.7 ppm, the content of protein: 3.0%). The resulting soybean milk pudding had light mouthfeel, and was easy to eat with reduced harsh taste and odor characteristic of soybean. Further, the bitter taste from 3"-α-GN was not sensed.

### (7) Orange jelly

Mixed were 20 parts by mass of powdered orange juice, 20 parts by mass of whey protein isolate (the content of protein: 85%), 0.8 parts by mass of agar, 0.2 parts by mass of locust bean gum, 35.5 parts by mass of dextrin, 22.0 parts by mass of powdered oils and fats, 0.5 parts by mass of salt, and 1.0 parts by mass of acidulant. To the resulting mixture, 200 parts by mass of water at 95°C was added, to which an enzyme-treated naringin composition (Product name "NARINVID" (registered trademark), the content of 3"-α-GN: 70%, available from Hayashibara Co., Ltd.) was then added to give a concentration of 10 ppm, and dissolved with stirring. The solution was filled into a container and then cooled to prepare orange jelly (the concentration of 3"-α-GN: 7 ppm, the content of protein: 5.7%). The resulting orange jelly had light mouthfeel, and was easy to eat with reduced milk odor characteristic of whey protein. Further, the bitter taste from 3"-α-GN was not sensed.

### (8) Soybean milk soup

Heated and stirred were 30 parts by mass of commercially available plain soybean milk (Product name: "Tokusei-Noukou 14.0 plain soybean milk", the content of soybean solid: 14%, the content of protein: 6.8%, available from MARUSAN-AI CO., LTD.), 60 parts by mass of water, 10 parts by mass of onion paste, 2 parts by mass of sugar, 0.5 parts by mass of salt, and emulsified at 10,000 rpm for 5 minutes. Then, an enzyme-treated naringin composition (Product name "NARINVID" (registered trademark), the content of 3"-α-GN: 70%, available from Hayashibara Co., Ltd.) was added to give a concentration of 5 ppm. Thereby, soybean milk onion soup (the content of 3"-α-GN: 3.5 ppm, the content of protein: 2%) was prepared. The resulting soybean milk onion soup had light mouthfeel, and was easy to eat with reduced harsh taste and soybean odor characteristic of soybean milk beverages. Further, the bitter taste from 3"-α-GN was not sensed.

### (9) Ice cream

Mixed were 110 parts by mass of sugar, 60 parts by mass of dextrin, 22 parts by mass of trehalose, 16 parts by mass of pea protein (the content of protein: 85%), 2 parts by mass of stabilizer, and 1.5 parts by mass of emulsifier, to which an enzyme-treated naringin composition (Product name "NARINVID" (registered trademark), the content of 3"-α-GN: 70%, available from Hayashibara Co., Ltd.) was added to give a concentration of 3 ppm. Further, 100 parts by mass of coconut oil, 60 parts by mass of starch syrup, and 603 parts by mass of water were added, and heat sterilized at 85°C while stirring and mixing, and then cooled through a homogenizer, and placed in an ice cream freezer to produce pea protein-containing ice cream (the content of 3"-α-GN: 2.1 ppm, the content of protein: 1.4%). The resulting ice cream had light mouthfeel, and was easy to eat with reduced harsh taste and bean odor characteristic of pea. Further, the bitter taste from 3"-α-GN was not sensed.

### (10) Chocolate protein bar

Mixed were 60 parts by mass of chocolate fat, 15 parts by mass of soybean puff (the content of protein: 79.9%), 10 parts by mass of a branched α-glucan mixture (Product name " FIBRYXA ", available from Hayashibara Co., Ltd.), 5 parts by mass of sugar, 5 parts by mass of trehalose (Product name "TREHA", available from Hayashibara Co., Ltd.), 5 parts by mass of separated soy protein (the content of protein: about 90%), 0.1 parts by mass of an enzyme-treated naringin composition (Product name "NARINVID" (registered trademark), the content of 3"-α-GN: 70%, available from Hayashibara Co., Ltd.), and an appropriate amount of a flavoring agent. Then, a chocolate protein bar with soybean puff (the content of 3"-α-GN: 7 ppm, the content of protein: 16.5%) were prepared according to a conventional method. The resulting protein bar had light mouthfeel although it was rich in protein, and was easy to eat with reduced harsh taste and odor characteristic of soybean. Further, the bitter taste from 3"-α-GN was not sensed.

### [Industrial applicability]

As described above, glucosyl naringin can improve the mouthfeel of protein-containing food materials. In particular, glucosyl naringin can effectively improve the mouthfeel of a protein-containing food material even at a low concentration within a range where the taste quality of glucosyl naringin itself does not make any impacts on the material. Therefore, even when a high concentration of a protein-containing material is included, the mouthfeel thereof can be lightened to provide a food and drink composition which is easy to eat and drink. The present invention can increase the variety of materials that can improve the mouthfeel of a protein-containing food material, and can increase the number of options in the production of food and drink compositions. Thereby, the present invention can expand the market of food and drink compositions comprising protein-containing food materials and thus can contribute to healthy lifestyles for people.

## Claims

1. A composition for improving a mouthfeel of a protein-containing food material, the composition comprising glucosyl naringin.

2. The composition for improving the mouthfeel according to claim 1, wherein the protein includes a plant or animal milk-derived protein.

3. A food and drink composition, comprising glucosyl naringin and a protein-containing food material.

4. The food and drink composition according to claim 3, comprising 0.5×10⁻⁶ to 1.0×10⁻² parts by mass of the glucosyl naringin relative to 1 part by mass of the protein.

5. The food and drink composition according to claim 3, wherein the protein includes a plant or animal milk-derived protein.

6. The food and drink composition according to claim 3, wherein the protein includes a legume milk-derived protein and/or a milk fluid-derived protein.

7. The food and drink composition according to claim 3, wherein the protein includes at least one selected from the group consisting of soybean protein, pea protein, whey protein, and casein.

8. The food and drink composition according to claim 3, wherein the protein includes a legume-derived protein.

9. The food and drink composition according to claim 3, which is in a liquid state, a fluidized state, a gel state, a semisolid state, a solid state, or a powdered state.

10. The food and drink composition according to claim 3, wherein the protein-containing food material includes soybean milk.

11. A method for manufacturing a food and drink composition according to any one of claims 3 to 10, the method comprising a step of mixing the protein-containing food material and glucosyl naringin.

12. The method for manufacturing according to claim 11, wherein 0.5×10⁻⁶ to 1.0×10⁻² parts by mass of the glucosyl naringin is blended relative to 1 part by mass of the protein.

13. A method of improving a mouthfeel of a protein-containing food material, the method comprising a step of blending glucosyl naringin with the protein-containing food material.

14. The method according to claim 13, wherein the protein includes a plant or animal milk-derived protein.

15. The method according to claim 13 or 14, wherein 0.5×10⁻⁶ to 1.0×10⁻² parts by mass of the glucosyl naringin is blended relative to 1 part by mass of the protein.
